# EUROPEAN PATENT APPLICATION

(11) **EP 2 062 498 A2**
(43) Date of publication of application: **27.05.2009**
(21) Application number: 08168971.3
(22) Date of filing: 12.11.2008
(51) Int. Cl.: A45D 44/00, A45D 44/08, A41D 13/04

(54) **Haircut Apron**

(30) Priority: 19.11.2007 GB 0722624; 10.04.2008 GB 0806514
(71) Applicant: Thesis Technology Products and Services Limited, Main Road Chichester, Sussex PO18 8AT (GB)
(72) Inventor: Palmer, Edward, Chichester, Sussex PO18 8AT (GB)
(74) Representative: Messulam, Adam Clive

(57) **Abstract**

A vest (10) having a head aperture (14) for placement around the neck of a wearer, wherein a circumferential seal (26) of flexible elastomeric waterproof material is welded to the head aperture, the vest further comprising an adjustable releasable fastener (32) secured to opposite sides of a substantially radial slit (30) extending through the seal, the slit and fastener enabling the seal to be adjusted and secured to accommodate wearers of differing neck circumference, characterised in that the circumferential seal is an annulus, welded to and in the same plane as the vest, and having a flexibility, resilience and width such that the seal self-conforms upwardly to the neck when fastened, forming a waterproof contact band.

## Description

The present invention relates to a waterproof and hair proof cover to protect the neck of a person during a hair cut. It may be used to protect medical dressings or venous lines, of medical patients, from water ingress.

At the present time, a medical dressing or venous line applied to the torso of a medical patient which is to be kept dry prevents the patient from effectively washing or taking a shower for fear of water coming into contact with the dressing or line and causing infection. As a result the dressing or line presents a problem to the satisfactory personal hygiene of the patient. The problem is exacerbated by the length of time that a dressing or venous line has to be kept in place to complete healing or enable treatment of the patient.

In the case of plaster casts and dressings applied to broken limbs, some effort has been made to alleviate the same problem by providing the patient with a bag of waterproof material to enclose the affected limb whilst the patient washes. The mouth of the bag is tied round the limb or secured with adhesive tapes in an effort to prevent wetting of the dressing or cast. This expedient has not proved satisfactory because of the failure of a tied or taped bag to keep out the water satisfactorily at the mouth of the bag. The bag material itself does not provide a satisfactory or consistent seal.

UK patent application GB2287194 solves this problem by providing a sealed waterproof tube having one open end comprising an elastic neoprene seal which secured around the limb and prevented the ingress of water. Though effective for the protection of limbs, such a solution is not applicable to the protection of the torso due to the need for movement of the patient and the need for the limbs to remain free.

Despite the very long standing nature of this problem and the large number of dressings and venous lines that are applied annually to medical patients, there has hitherto been no satisfactory solution proposed to meet the problem of keeping such dressings and venous lines dry whilst the patient is allowed to wash or bathe.

In addition to providing the solution, the present invention is intended to solve a less serious though far more widespread problem relating to hair dressing. Hair dressers often provide some form of means for preventing the ingress of hair down the neck of customers during a haircut. This problem is reduced by wetting the hair causing it clump together, this also aids styling and cutting. In addition to this, many salons provide gowns with a draw string loosely tied around the neck, as well as towels and rubberised mats which sit around the neck holding the gown in place and attempting to provide some degree of seal around the neck line. These are often heavy and cumbersome, but more importantly, predominantly ineffective.

There is now provided in accordance with this invention a vest having a head aperture for placement around the neck of a wearer, wherein a circumferential seal of flexible elastomeric waterproof material is welded to the head aperture, the vest further comprising an adjustable releasable fastener secured to opposite sides of a substantially radial slit extending through the seal, the slit and fastener enabling the seal to be adjusted and secured to accommodate wearers of differing neck circumference, characterised in that the circumferential seal is an annulus, welded to and in the same plane as the vest, and having a flexibility, resilience and width such that the seal self-conforms upwardly to the neck when fastened, forming a waterproof contact band.

Preferably the elastomeric waterproof seal may be coated in a sealant to prevent hair cuttings from penetrating the mesh of the elastomeric material. The sealant may be naphtha based.

Additionally, the sealant may have anti-static and/or low friction properties.

Advantageously the vest is made of a waterproof material.

Preferably the seal may be formed of a butyl, nitrile, ethylene propyl diomonomer, or latex rubber.

Conveniently the cover may be formed of a polyethylene, a polypropylene, a polyester, a polyurethane, or a polyvinyl chloride material.

Advantageously a front and rear face of the vest are joined to one another to form a waist band and arm apertures for receiving the arms of a patient.

The arm apertures may additionally be provided with elastomeric waterproof seals to prevent ingress of water to the chest area of a patient.

The slit may extend from the inner to the outer circumference of the seal.

The slit may further extend substantially radially beyond the outer circumference of the seal into the vest to allow for greater adjustment range of the neck aperture circumference, or alternatively it may extend to the outer edge of the vest to allow the vest wrap around the neck without disturbing the hair.

Preferably the vest has a front face and a rear face, and the slit is provided in the front face of the seal (and vest) such that when in use, the slit is as remote as possible from the hairline of a user.

Preferably the adjustable releasable fastener may be a hook and loop fastener, a button and hole fastener, a press stud fastener or strap and buckle.

The invention will now be described with reference to the accompanying drawings in which:
Figure 1 is a top view of the vest according to a preferred embodiment of the present invention,
Figure 2 is a rear view of a patient wearing the vest according to a medical embodiment of the present invention, and
Figure 3 is a front view of a patient wearing the vest according to a medical embodiment of the present invention.

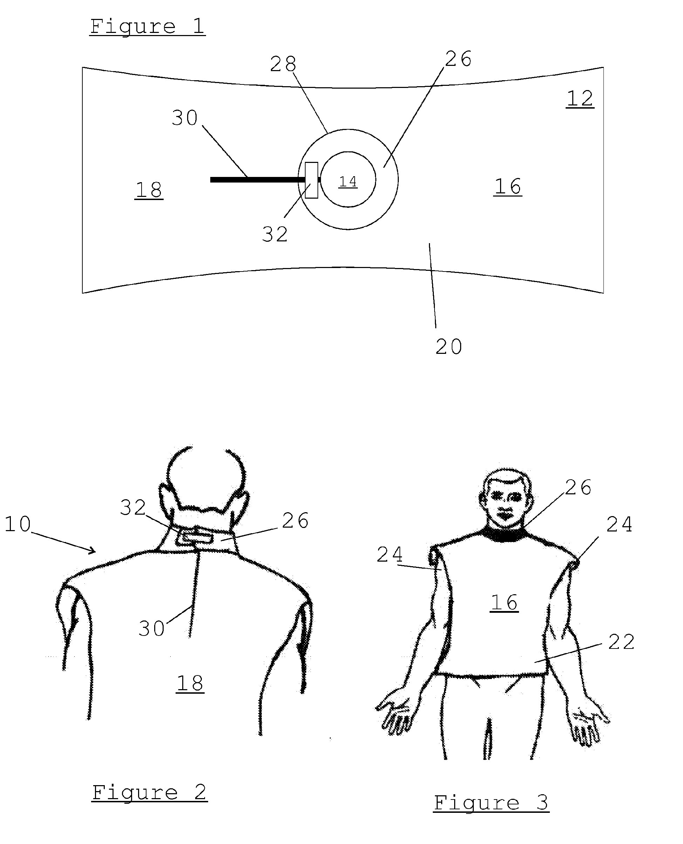

Referring to Figure 1 there is shown a vest 10 be fitted over a medical dressing on or venous line in the chest of a patient. Alternatively, the vest can be used as a shawl to shroud the clothing of a person having their hair cut, the vest being used to keep cut hair away off the clothes and most importantly, the neck. In such a circumstance, the vest need not be waterproof.

The vest is made as a flat blank 12 as shown in figure 1, having a central head aperture 14 for receiving the head of a patient when in use. The blank 12 is folded in half about a line through the head aperture 14 forming a front face 16 and a rear face 18.

The front and rear faces form the cover portion 20 of the vest 10. The front and rear faces 16 and 18 may be joined by plastic welding or bonding at the waist band 22, to prevent the cover portion 20 from flapping. In so doing, arm apertures 24 are formed in the cover to create a vest 10 as shown in figures 2 and 3.

It should be noted that for the purposes of this patent specification the term vest is intended to refer to a protective garment which may include a poncho which merely fits over the head of a patient, or a typical vest type garment having apertures for the torso and arms. Additionally it is intended to cover a vest having a slit extending from the neck aperture to the outer edge of the vest allowing the vest to be wrapped around the neck without placing it over the head of a its wearer.

For medical purposes it may be considered desirable to allow the patient as much freedom of movement as possible, and therefore, the open poncho design of figure 1 is advantageous. This is also preferential when used in hair dressing purposes.

The cover portion 20 may be of any suitable flexible material (waterproof or otherwise) such as a synthetic polymeric material. A polyethylene, a polypropylene, a polyester, a polyurethane, a polyvinyl chloride with or without a reinforcing material such as nylon may alternatively be used.

Returning to figure 1, the head aperture 14 is joined about its entire circumference to a seal 26. The seal 26 is formed from an annulus the central aperture of which is intended to receive the head. The periphery of the annulus is (preferably high frequency)welded to the cover portion 20 at a join 28. Welding provides advantages over bonding in that it is more consistent during manufacture, stronger and cheaper without adding uncomfortable bulk in the sensitive area of the neck.

The seal 26 is made of a flexible elastomeric waterproof material which is soft and compliant to the skin of the neck. The elastomeric material may be a synthetic or natural rubber and may in particular be a polymer or copolymer of chloroprene. The seal is arranged in the same plane as the vest to facilitate ease of welding to the vest.

The seal therefore constitutes a band of material which can be stretched to open the head aperture 16 to receive the head of a patient. The disc can thereafter be allowed to contract to press lightly against the skin of the neck once the vest 10 has been drawn into position shrouding the torso. Since the seal is annular in shape it has a thickness, and when combined with the resilience and flexibility of the material from which the seal is made, conforms to the shape of the neck, usually by bending upwards to form a band seal around the neck rather than a line seal. This results in making the vest considerably more comfortable to wear whilst still keeping out water or cut hair.

The crucial difference this provides over the prior art, particularly in relation to its use in hair cutting, is that the seal of the present invention conforms to the neckline by deflecting upwardly around the neck to form a band contact. This is only possible because the present invention provides an annulus of elastomeric material, having a substantial radius, allowing a greater width of resilient material to conform to the neck rather than a ring of elastic only sufficient to form a line seal around the neck, as shown in the prior art.

This has the further advantage that it can be partially pulled away from the neck to expose areas of the hairline for cutting or sweeping out of stray hairs that may have become trapped, without completely opening up the band seal, since there is sufficient axial length of circumferential seal to remain in contact with the neck.

Additionally, since the seal is made of a waterproof material, it has the added benefit of guarding the visible skin of the neck against shampoo that may irritate and against hair dye that may leave visible stains.

To further extend the comfort of a wearer of the vest, the elastomeric seal may be coated using a suitable sealant. One such example is Fabsil_{TM}. This is a naphtha based product which is sprayed onto sheets of the elastomeric material before it is cut to the correct shape and size. Additionally silicon based coatings may be used.

The coating provides the advantage of preventing hair cuttings from becoming lodged in the mesh of the elastomeric seal, making the seal more comfortable. Additionally, the coating may have anti-static and anti-friction properties, intended to encourage hair to fall onto a gown (worn underneath the vest) or onto the floor. It is disadvantageous for any amount of hair to stick to seal, either mechanically or statically, since any hair can be irritating to the wearer, but perhaps more importantly, unwanted hair trapped within the elastomeric seals makes it far more difficult to be removed quickly, between hair cutting appointments.

In order to accommodate patients of varying neck size, the seal 26 and cover portion 20 are provided with a slit 30 that runs substantially radially outward from the inner circumferential edge of the seal 26 down the rear face 18 of the cover portion 20 as shown clearly in figure 2. It may further extend to the outer edge of the vest to avoid the need to place the vest over the head, as mentioned earlier.

An adjustable releasable fastener 32 is secured to the seal 26 either side of the slit 30. This allows the head aperture 14 to be opened to suit differing sizes of head and neck. The fastener 32 then secures the seal around the neck of the patient at the desired level of tightness.

The adjustable releasable fastener 32 may be any suitable type e.g. a hook and loop fastener, a buckle, button and hole.

When used to protect medical dressings, the vest is positioned so that the slit 30 is located on the rear face of the seal 26 and the cover 20, such that it is shielded by the neck of the patient against splashing when the vest 10 is in place. This means the important area to be protected from the ingress of water, namely the chest area, is protected by an unbroken seal 26 around the front of the neck.

When used for preventing hair from falling down the neck, it is preferable for vest to be positioned so that the slit 30 to be located at the front, under the chin area of its user.

The advantage that has been discovered with the arrangement as shown and described in the figures is that a seal can be provided by means of the seal 26 whilst the pressure exerted against the skin is not sufficient to cause harm to the circulation of the user to cause any other deleterious effects.

In alternative embodiments relating to protecting medical dressings and venous lines, for additional protection from water, similar seals can be provided about the arm apertures 24.

## Claims

1. A vest having a head aperture for placement around the neck of a wearer, wherein a circumferential seal of flexible elastomeric waterproof material is welded to the head aperture, the vest further comprising an adjustable releasable fastener secured to opposite sides of a substantially radial slit extending through the seal, the slit and fastener enabling the seal to be adjusted and secured to accommodate wearers of differing neck circumference,
**characterised in that** the circumferential seal is an annulus, welded to and in the same plane as the vest, and having a flexibility, resilience and width such that the seal self-conforms upwardly to the neck when fastened, forming a waterproof contact band.

2. A vest as claimed in claim 1, where the elastomeric waterproof seal is coated in a sealant to prevent hair cuttings from penetrating the mesh of the elastomeric material.

3. A vest as claimed in claim 2, wherein the sealant is naphtha based.

4. A vest as claimed in claim 2 or 3, wherein the sealant has at least one of anti-static or low friction properties.

5. A vest as claimed in claims 1 to 4, wherein the vest is made of a waterproof material.

6. A vest as claimed in any preceding claim, wherein the seal is formed of a butyl, nitrile, ethylene propyl diomonomer, or latex rubber.

7. A vest as claimed in any preceding claim, wherein the cover is formed of a polyethylene, a polypropylene, a polyester, a polyurethane, or a polyvinyl chloride material.

8. A vest as claimed in any preceding claim, wherein a front and rear face of the vest are joined to one another to form a waist band and arm apertures for receiving the arms of a patient.

9. A vest as claimed in claim 8, wherein the arm apertures are also provided with elastomeric waterproof seals to prevent ingress of water to the chest area of a patient.

10. A vest as claimed in any preceding claim, wherein the slit extends from the inner to the outer circumference of the seal.

11. A vest as claimed in claim 10, wherein the slit further extends substantially radially beyond the outer circumference of the seal into the vest to allow for greater adjustment range of the neck aperture circumference.

12. A vest as claimed in any preceding claim, wherein the vest has a front face and a rear face, and the slit is provided in the rear face of the seal such that when in use, the slit is as remote as possible from the chest area of a patient.

13. A vest as claimed in any preceding claim, wherein the adjustable releasable fastener is a hook and loop fastener.

14. A vest as claimed in claims 1 to 12, wherein the adjustable releasable fastener is at least one of a button and hole fastener, a press stud fastener and a buckle.
